(19) Europäisches Patentamt
European Patent Office
Office européen des brevets

(11) **EP 4 272 904 A1**

(12) **EUROPEAN PATENT APPLICATION**
published in accordance with Art. 153(4) EPC

(43) Date of publication:
**08.11.2023 Bulletin 2023/45**

(21) Application number: **20967505.7**

(22) Date of filing: **30.12.2020**

(51) International Patent Classification (IPC):
**B25J 9/00** (2006.01)

(52) Cooperative Patent Classification (CPC):
**B25J 9/00**

(86) International application number:
**PCT/CN2020/141274**

(87) International publication number:
**WO 2022/141160 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Noahtron Intelligence Medtech (Hangzhou) Co., Ltd.**
**Hangzhou, Zhejiang 310051 (CN)**

(72) Inventors:
• **HUANG, Shandeng**
**Hangzhou, Zhejiang 310051 (CN)**

• **BAI, Long**
**Hangzhou, Zhejiang 310051 (CN)**
• **CHEN, Xiaohong**
**Hangzhou, Zhejiang 310051 (CN)**
• **PAN, Lufeng**
**Hangzhou, Zhejiang 310051 (CN)**
• **LIU, Jianfei**
**Hangzhou, Zhejiang 310051 (CN)**

(74) Representative: **Petraz, Gilberto Luigi et al**
**GLP S.r.l.**
**Viale Europa Unita, 171**
**33100 Udine (IT)**

(54) **MASTER-SLAVE MAPPING METHOD FOR PARALLEL PLATFORM, AND MECHANICAL ARM SYSTEM AND STORAGE MEDIUM**

(57)    The present application relates to a master-slave mapping method for parallel platform, and a robotic arm system and a storage medium. The method comprises: acquiring a first transformation relationship between a user coordinate system and a calculation coordinate system; mapping displacement amounts of an end of a master manipulator in a master user coordinate system to an end of the movable platform according to a set proportional coefficient, to obtain a first target position of the end of the movable platform; determining a second target position of the end of the movable platform according to the first transformation relationship and the first target position; obtaining a movement amount of each of the telescopic rods of the parallel platform according to the second target position, and controlling a motion of the parallel platform according to the movement amount of each of the telescopic rods. The control of the robotic arm is simplified.

establishing the calculation coordinate system of the parallel platform on the static platform, establishing the slave user coordinate system on the static platform, and establishing the master user coordinate system on the master manipulator — S101

acquiring a first transformation relationship between the slave user coordinate system and the calculation coordinate system — S102

mapping the displacement amount of an end of the master manipulator in the master user coordinate system to an end of the movable platform in the slave user coordinate system according to a set proportional coefficient, to obtain the first target position of the end of the movable platform in the slave user coordinate system — S103

determining the second target position of the end of the movable platform in the calculation coordinate system according to the first transformation relationship and the first target position — S104

obtaining a movement amount of each of the telescopic rods of the parallel platform by using inverse kinematics algorithm according to the second target position, and controlling a motion of the parallel platform according to the movement amount of each of the telescopic rods — S105

**FIG. 1**

**Description**

TECHNICAL FIELD

The present application relates to the field of control, and in particular to a master-slave mapping method for parallel platform, a robotic arm system and a storage medium.

DESCRIPTION OF THE PRIOR ART

To realize the master-slave control of a robotic arm, it is necessary to solve the kinematics of a master manipulator, the kinematics of a slave manipulator, and a motion mapping algorithm from the master manipulator to the slave manipulator. While the kinematics solution depends on the establishment of a coordinate system, and the way of establishing the coordinate system will affect the complexity of the kinematics algorithm.

SUMMARY OF THE DISCLOSURE

According to various embodiments of the present application, a master-slave mapping method for parallel platform is provided, which is applied to a robotic arm system. The robotic arm system includes a parallel platform and a master manipulator, and the parallel platform includes a static platform, a movable platform, and a plurality of telescopic rods arranged between the static platform and the movable platform. The master-slave mapping method for parallel platform includes: establishing a calculation coordinate system of the parallel platform on the static platform, establishing a slave user coordinate system on the static platform and a master user coordinate system on the master manipulator, wherein the origin of the calculation coordinate system coincides with the origin of the slave user coordinate system, and the Z axes of the slave user coordinate system and the master user coordinate system are both parallel to the Z axis of a reference coordinate system; acquiring a first transformation relationship between the slave user coordinate system and the calculation coordinate system; mapping displacement amounts of an end of the master manipulator in the master user coordinate system to displacement amounts of an end of the movable platform in the slave user coordinate system according to a set proportional coefficient to obtain a first target position of the end of the movable platform in the slave user coordinate system; determining a second target position of the end of the movable platform in the calculation coordinate system according to the first transformation relationship and the first target position; obtaining a movement amount of each of the telescopic rods of the parallel platform by using inverse kinematics algorithm according to the second target position, and controlling a motion of the parallel platform according to the movement amount of each of the telescopic rods.

The above-mentioned master-slave mapping method for parallel platform has the following advantages: compared with the related art, the master-slave mapping method for parallel platform, the robotic arm system and the storage medium provided by the embodiments of the present application can solve the problem that the control of a robotic arm is complicated in the related art, reducing the control complexity of the robotic arm.

The parallel platform according to the embodiments can achieve motions with multiple degrees of freedom. Taking the Stewart parallel platform with six degrees of freedom as an example, the Stewart parallel platform include a static platform, a movable platform, and a plurality of telescopic assemblies arranged between the static platform and the movable platform, which can achieve motions with six degrees of freedom in space, namely displacement along the X axis, displacement along the Y axis, displacement along the Z axis, rotation about the X axis, rotation about the Y axis, and rotation about the Z axis. The Stewart parallel platform are supported by 6 telescopic assemblies, which has a higher rigidity with a structure more stable than the slave manipulator using a structure of cantilever beam structure in series. Due to the higher rigidity, the parallel structure has a higher bearing capacity than a series structure under the same self-weight or volume. The error at the end of the slave manipulator using the cantilever beam structure in series is the accumulation and amplification of the errors at each joint, therefore the error is large and the precision is low. The parallel platform does not have such error accumulation and amplification relationship; resulting in high the micro-motion accuracy, and thus is more suitable for high-precision surgical operations. In addition, in terms of position solution, the inverse solution of the parallel platform is very easy, and it is easy to obtain the motion posture of each telescopic assembly of the parallel platform according to the coordinate position.

It is because of the basis on the characteristics that the inverse solution of the parallel platform is very easy, that in the above steps, by establishing the master user coordinate system and the slave user coordinate system, the control amount of the master manipulator in the master user coordinate system is mapped into the slave user coordinate system in the form of displacement amount. Then the position information of the second target position in the calculation coordinate system can be obtained through the transformation between the slave user coordinate system and the calculation coordinate system. According to this position information, the motion posture of each telescopic assembly of the parallel platform can be easily obtained through inverse solution. Compared with the position information of the end of the parallel platform solved by direct kinematics in related art, the above method greatly reduces the complexity of computation, improves the control efficiency, and saves computing resources.

In some of the embodiments, the step of acquiring a first transformation relationship between the slave user coordinate system and the calculation coordinate system includes: acquiring a second transformation relationship between the reference coordinate system and the slave user coordinate system, and acquiring a third transformation relationship between the reference coordinate system and the calculation coordinate system; and determining the first transformation relationship according to the second transformation relationship and the third transformation relationship.

In some of the embodiments, the reference coordinate system is the base coordinate system of the robotic arm system. The robotic arm system further includes a serial robotic arm, and the parallel platform is mounted on an end of the serial robotic arm, wherein, acquiring the third transformation relationship between the reference coordinate system and the calculation coordinate system includes: establishing a joint coordinate system of each joint in the serial robotic arm; acquiring DH parameters of joint coordinate system of the first joint in the serial robotic arm and the reference coordinate system and determining the transformation relationship $_{1}^{0}T$ between the reference coordinate system and the joint coordinate system of a first joint, wherein the first joint is a joint directly connected to the base, and the DH parameters are traditional DH parameters or improved DH parameters; acquiring the DH parameters of the joint coordinate system of the i-th joint and the joint coordinate system of the (i-1)-th joint in the serial robotic arm, and determining the transformation relationship $_{i}^{i-1}T$ between the joint coordinate system of the (i-1)-th joint and the joint coordinate system of the i-th joint, where i=2, 3, 4,...,N; N is the total number of joints of the serial robotic arm; determining the transformation relationship $_{N}^{0}T$ between the reference coordinate system and the joint coordinate system of the N-th joint according to the transformation relationship $_{1}^{0}T$ and the transformation relationship $_{i}^{i-1}T$ , which is the third transformation relationship, wherein the joint coordinate system of the N-th joint coincides completely with the calculation coordinate system.

In some of the embodiments, the serial robotic arm includes rotating joints and translating joints. Wherein the Z axis of the joint coordinate system of the rotating joint is set along the rotation axis, and the Z axis of the joint coordinate system of the translating joint is set along the moving direction. The reference coordinate system and the joint coordinate systems of each joint are all left-handed system or right-handed system, and when the joint before the rotating joint is a translating joint, the origin of the joint coordinate system of the rotating joint coincides with the origin of the joint coordinate system the translating joint.

In some of the embodiments, the rotation angle of the Z axis in the DH parameters of the rotating joint in the serial robotic arm is not 0 or $2\pi$.

In some of the embodiments, acquiring the second transformation relationship between the reference coordinate system and the slave user coordinate system includes: acquiring a viewing angle value of the user relative to the robotic arm system; and determining the second transformation relationship between the reference coordinate system and the slave user coordinate system according to the viewing angle value and the third transformation relationship.

In some of the embodiments, the viewing angle value is determined based on configuration information input by the user in the case the the robotic arm system operates with a single arm.

In some of the embodiments, in the case of a first serial robotic arm and a second serial robotic arm working simultaneously in the robotic system, acquiring the second transformation relationship between the reference coordinate system and the slave user coordinate system includes: establishing a first slave user coordinate system of the first serial robotic arm and a second slave user coordinate system of the second serial robotic arm, wherein the X-axis direction of the first slave user coordinate system is the same as and collinear with the X-axis direction of the second slave user coordinate system, and the origin of the first slave user coordinate system coincides with the origin of the calculation coordinate system of the first serial robotic arm, and the origin of the second slave user coordinate system coincides with the origin of the calculation coordinate system of the second serial robotic arm; determining the angle between the X-axis direction of the first slave user coordinate system and the X-axis direction of the reference coordinate system, and determining the second transformation relationship between the reference coordinate system and the first slave user coordinate system and the second transformation relationship between the reference coordinate system and the second slave user coordinate system according to the angle and transformation relationship between the reference coordinate system and the calculation coordinate system of each serial robotic arm.

According to various embodiments of the present application, a robotic arm system is provided, which includes a parallel platform, a master manipulator, a memory, and a controller. The parallel platform includes a static platform, a movable platform, and a plurality of telescopic rods arranged between the static platform and the movable platforms. A computer program is stored in the memory, and the controller is configured to run the computer program to execute the master-slave mapping method for parallel platform provided by the embodiments of the present application.

In some of the embodiments, the robotic arm system further includes a serial robotic arm, and the parallel platform is mounted on an end of the serial robotic arm.

According to various embodiments of the present application, a storage medium is provided, on which a computer program is stored, wherein the computer program is configured to execute the master-slave mapping method for parallel platform provided by the embodiments of the present application when running.

BRIEF DESCRIPTION OF DRAWINGS

In order to better describe and illustrate embodiments and/or examples of those inventions disclosed herein, reference may be made to one or more of the accompanying drawings. The additional details or examples used to describe the drawings should not be construed as limiting the scope of any of the disclosed inventions, the presently described embodiments and/or examples, and the best modes presently understood of these inventions.

FIG. 1 is a flowchart of a master-slave mapping method for parallel platform according to an embodiment of the present application.

FIG. 2 is a flowchart of a coordinate transformation method according to an embodiment of the present application.

FIG. 3 is a schematic structural view of a robotic arm system provided by an optional embodiment of the present application.

FIG. 4 is a schematic diagram illustrating a reference coordinate system and a joint coordinate system of a robotic arm system provided by an optional embodiment of the present application.

FIG. 5 is a schematic diagram of the slave user coordinate system when two arms of the robotic arm system provided by the optional embodiment of the present application work.

FIG. 6 is a schematic diagram of a master-slave mapping method for parallel platform according to an optional embodiment of the present application.

DESCRIPTION OF EMBODIMENTS

The master-slave mapping method for parallel platform, robotic arm system and storage medium provided by the present application will be further described as follows.

This embodiment provides a master-slave mapping method for parallel platform. The master-slave mapping method for parallel platform is applied to a robotic arm system including a parallel platform and a master manipulator. The parallel platform includes a static platform, a movable platform and a plurality of telescopic rods arranged between the static platform and the movable platform.

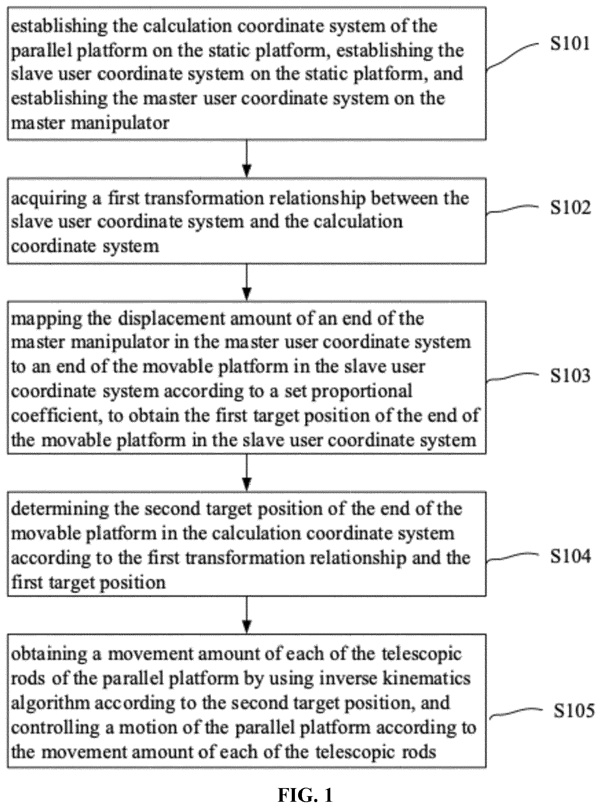

FIG. 1 is a flowchart of a master-slave mapping method for a parallel platform according to an embodiment of the present application. As shown in FIG. 1 the flowchart includes the following steps.

Step S101: establishing the calculation coordinate system of the parallel platform on the static platform, establishing the slave user coordinate system on the static platform, and establishing the master user coordinate system on the master manipulator, wherein the origin of the calculation coordinate system coincides with the origin of the slave user coordinate system, and the Z axes of the slave user coordinate system and the master user coordinate system are both parallel to the Z-axis of the reference coordinate system.

Step S102: acquiring a first transformation relationship between the slave user coordinate system and the calculation coordinate system.

Step S103: mapping the displacement amount of an end of the master manipulator in the master user coordinate system to the displacement amount of an end of the movable platform in the slave user coordinate system according to a set proportional coefficient, to obtain the first target position of the end of the movable platform in the slave user coordinate system.

Step S104: determining the second target position of the end of the movable platform in the calculation coordinate system according to the first transformation relationship and the first target position.

Step S105: obtaining a movement amount of each of the telescopic rods of the parallel platform by using inverse kinematics algorithm according to the second target position, and controlling a motion of the parallel platform according to the movement amount of each of the telescopic rods.

The parallel platform in this embodiment can achieve motions with multiple degrees of freedom. Taking the Stewart parallel platform with six degrees of freedom as an example, the Stewart parallel platform includes a static platform, a movable platform, and a plurality of telescopic assemblies arranged between the static platform and the movable platform, which can achieve motions with six degrees of freedom in space, namely displacement along the X axis, displacement along the Y axis, displacement along the Z axis, rotation about the X axis, rotation about the Y axis, and rotation about the Z axis. The Stewart parallel platform is supported by 6 telescopic assemblies, which has a higher rigidity with a structure more stable than the slave manipulator using a structure of cantilever beam structure in series.

Due to the higher rigidity, the parallel structure has a higher bearing capacity than a series structure under the same self-weight or volume. The error at the end of the slave manipulator using the structure with cantilever beam structure in series is the accumulation and amplification of the errors at each joint, therefore the error is large and the precision is low. The parallel platform does not have such error accumulation and amplification relationship, resulting in high micro-motion accuracy, and thus is more suitable for high-precision surgical operations. In addition, in terms of position solution, the inverse solution of the parallel platform is very easy, and it is easy to obtain the motion posture of each telescopic assembly of the parallel platform according to the coordinate position.

It is because of the basis of the characteristics that the inverse solution of the parallel platform is very easy, that in the above steps, by establishing the master user coordinate system and the slave user coordinate system, the control amount of the master manipulator in the master user coordinate system is mapped into the slave user coordinate system in the form of displacement amount. Then the position information of the second target position in the calculation coordinate system can be obtained through the transformation between the slave user coordinate system and the calculation coordinate system. According to this position information, the motion posture of each telescopic assembly of the parallel platform can be easily obtained through inverse solution. Compared with the position information of the end of the parallel platform solved by direct kinematics in related art, the above method greatly reduces the complexity of computation, improves the control efficiency, and saves computing resources.

The above-mentioned reference coordinate system is also called the global coordinate system, and said coordinate system can be selected arbitrarily. However, in order to make the calculation more convenient, the base coordinate system established at the center of the base bottom of the robotic arm system is selected as the reference coordinate system in the embodiment of the present application.

It should be noted that, in the above embodiments, the master user coordinate system and the slave user coordinate system may be the same coordinate system, or may be different coordinate systems. In case that they are the same coordinate system, that is to say, the origins of the master user coordinate system and the slave user coordinate system, and corresponding coordinate axes are completely coincident, that is, the coordinate origin of the master user coordinate system also coincides with the coordinate origin of the calculation coordinate system on the static platform. In case that they are different coordinate systems, the coordinate origin of the master user coordinate system for example, can be set at the end of the master manipulator, and each coordinate axis of the master user coordinate system is parallel to a corresponding coordinate axis of the slave user coordinate system, so as to facilitate the calculation.

In some of the embodiments, the first transformation relationship between the slave user coordinate system and the calculation coordinate system can be obtained by the following methods: acquiring a second transformation relationship between the reference coordinate system and the slave user coordinate system, and acquiring a third transformation relationship between the reference coordinate system and the calculation coordinate system; and determining the first transformation relationship according to the second transformation relationship and the third transformation relationship.

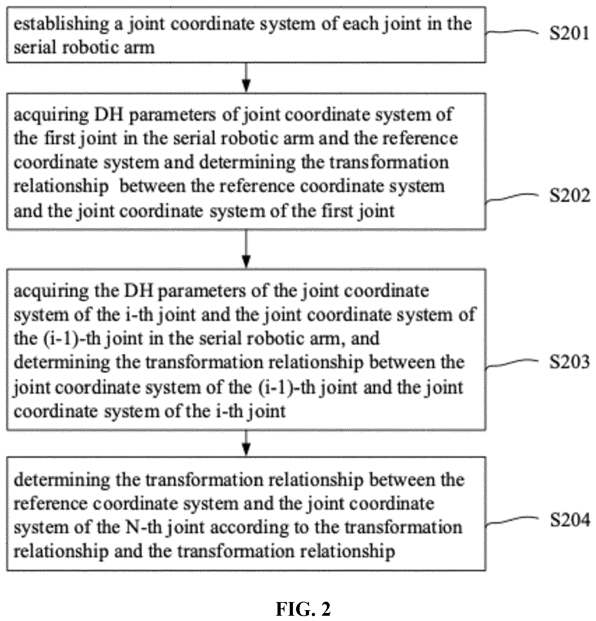

In some of the embodiments, the reference coordinate system is the base coordinate system of the robotic arm system. The robotic arm system further includes a serial robotic arm, and the parallel platform is mounted on the end of the serial robotic arm. FIG. 2 is a coordinate transformation method according to an embodiment of the present application. As shown in FIG. 2, acquiring the third transformation relationship between the reference coordinate system and the calculation coordinate system includes the following steps.

Step S201: establishing a joint coordinate system of each joint in the serial robotic arm.

The control of serial robotic arm is usually based on DH parameters or improved DH parameters for coordinate system transformation. Two joints connected each other are adjacent joints. The transformation of the joint coordinate system of two adjacent joints is usually represented by DH parameters or improved DH parameters. Taking the DH parameters as an example, two adjacent joint coordinate systems can coincide with each other by rotating by $\theta$ about the Z axis and translating by d, and then rotating by $\alpha$ around the X axis and translating by a. The above $\theta$, d, $\alpha$ and a are the DH parameters. It can be seen from this that the simpler the DH parameters are, the simpler the transformation of two adjacent joint coordinate systems will be.

In order to simplify the DH parameters, in this embodiment, the Z axis of the joint coordinate system of the rotating joint is set along the rotation axis, the Z axis of the joint coordinate system of the translating joint is set along the moving direction, and the reference coordinate system and the joint coordinate system of each joint are all left-handed or right-handed systems. When the joint before the rotating joint is a translating joint, the origin of the joint coordinate system of the rotating joint coincides with the origin of the joint coordinate system of the translating joint.

In a serial robotic arm, in most cases, when a rotating joint receives a command with a rotation angle of 0 or $2\pi$, it may be unnecessary to distinguish between the two angles, but it will not rotate or rotate by $2\pi$ in a set direction according to preset settings. However, in some cases, it is necessary to distinguish between these two rotation angles. In the case where the 0 or $2\pi$ rotation angle needs to be distinguished, the rotation angle of the Z axis in the DH parameters of the rotating joint is not 0 or $2\pi$, so as to avoid confusion between the rotation angle of 0 or $2\pi$.

Step S202: acquiring DH parameters of joint coordinate system of the first joint in the serial robotic arm and the reference coordinate system and determining the transformation relationship $^0_1T$ between the reference coordinate system and the joint coordinate system of the first j oint, wherein the first j oint is a j oint directly connected to the base, and the DH parameters are traditional DH parameters or improved DH parameters.

Step S203: acquiring the DH parameters of the joint coordinate system of the i-th joint and the joint coordinate system of the (i-1)-th joint in the serial robotic arm, and determining the transformation relationship $^{i-1}_iT$ between the joint coordinate system of the (i-1)-th joint and the joint coordinate system of the i-th joint, where i=2, 3, 4,...,N; N is the total number of joints of the serial robotic arm.

Step S204: determining the transformation relationship $^0_NT$ between the reference coordinate system and the joint coordinate system of the N-th joint according to the transformation relationship $^0_1T$ and the transformation relationship $^{i-1}_iT$, which is the third transformation relationship, wherein the joint coordinate system of the N-th joint coincides completely with the calculation coordinate system.

After the joint coordinate system of each joint is established, the DH parameters between adjacent joints can be obtained in turn, and the transformation relationship between the reference coordinate system of the robotic arm system and the calculation coordinate system of the serial robotic arm can be determined according to the DH parameters.

According to the DH rules, the homogeneous transformation from the coordinates of the (i-1)-th joint to the coordinates of the i-th joint is constructed as a sequence with two rotations and two transformations, which can be expressed as follows using a matrix:

$$^{i-1}_iT = \begin{bmatrix} \cos\theta_i & -\sin\theta_i & 0 & a_{i-1} \\ \sin\theta_i\times\cos\alpha_{i-1} & \cos\theta_i\times\cos\alpha_{i-1} & -\sin\alpha_{i-1} & -d_i\times\sin\alpha_{i-1} \\ \sin\alpha_{i-1}\times\sin\theta_i & \sin\alpha_{i-1}\times\cos\theta_i & \cos\alpha_{i-1} & d_i\times\cos\alpha_{i-1} \\ 0 & 0 & 0 & 1 \end{bmatrix}$$

Where, i=2,3,4,...,N; N is the total number of rotating joints and translating joints of the serial robotic arm. The DH parameters of the first joint of the serial robotic arm represent the transformation of the coordinate system between the first joint and the reference coordinate system, which is denoted as $^0_1T$, then the transformation between the reference coordinate system and the joint coordinate system of the first joint relation is:

$$^0_1T = \begin{bmatrix} \cos\theta_1 & -\sin\theta_1 & 0 & 0 \\ \sin\theta_1\times\cos\alpha_0 & \cos\theta_1\times\cos\alpha_0 & -\sin\alpha_0 & -d_1\times\sin\alpha_0 \\ \sin\alpha_0\times\sin\theta_1 & \sin\alpha_0\times\cos\theta_1 & \cos\alpha_0 & d_1\times\cos\alpha_0 \\ 0 & 0 & 0 & 1 \end{bmatrix}$$

It can be seen that the transformation relationship is exactly the same as the homogeneous transformation form from the coordinates of the (i-1)-th joint to the coordinates of the i-th joint.

After obtaining $^0_1T$ and $^{i-1}_iT$, the transformation matrix from the 0-th coordinate system (reference coordinate system) to the joint coordinate system of the N-th joint can be expressed as:

$$^0_NT = {}^0_1T \cdot {}^1_2T \cdots {}^{N-2}_{N-1}T \cdot {}^{N-1}_NT$$

Where, the N-th joint is an end joint.

The determined $^0_NT$ in the above step S204 represents the coordinate transformation relationship between the reference coordinate system and the calculation coordinate system, and the coordinate transformation between the reference coordinate system and the calculation coordinate system can be realized according to the transformation relationship.

The embodiments of the present application will be described and illustrated below through optional embodiments.

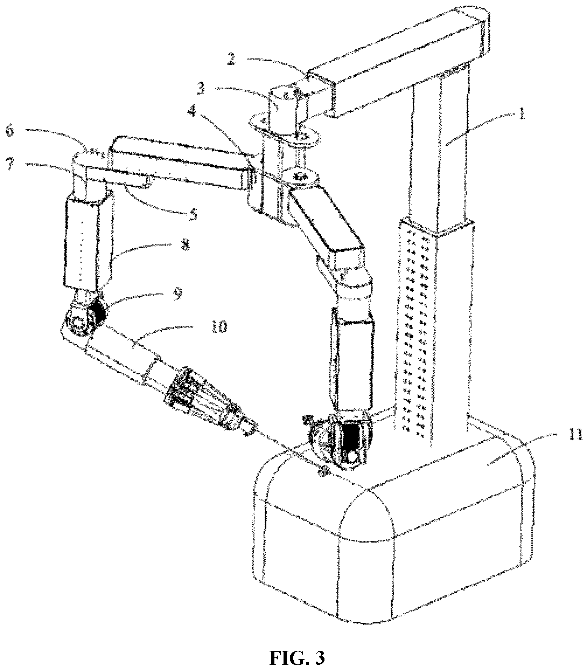

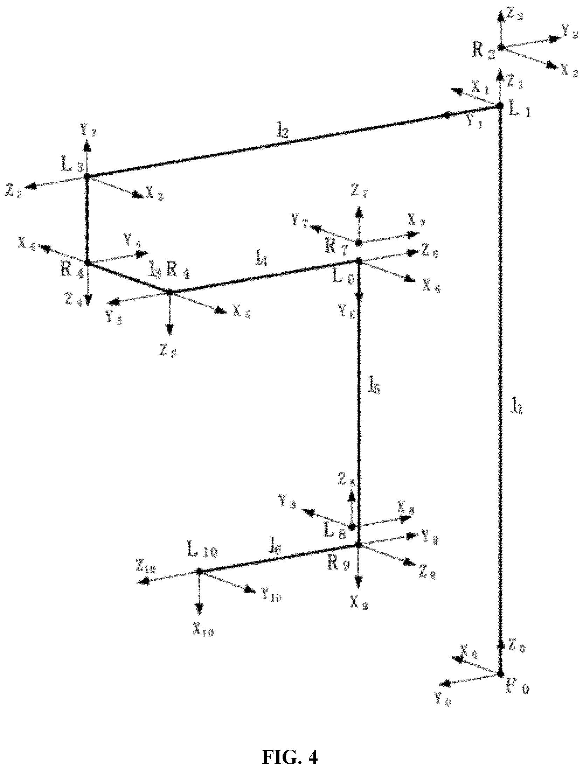

FIG. 3 is a schematic structural view of a robotic arm system provided by an optional embodiment of the present application. The robotic arm system shown in FIG. 3 includes a serial robotic arm, and the serial robotic arm includes a translating joint 1, a rotating joint 2, a translating joint 3, a rotating joint 4, a rotating joint 5, a translating joint 6, a rotating joint 7, a translating joint 8, a rotating joint 9 and a translating joint 10. In addition, the robotic arm system further includes a base 11 fixedly connected with the translating joint 1. FIG. 4 is a schematic diagram illustrating a reference coordinate system and a joint coordinate system of a robotic arm system provided by an optional embodiment of the present application. Referring to FIG. 4, the master-slave mapping method for parallel platform of this optional embodiment includes the following steps.

Step 1: establishing a reference coordinate system at the base of the serial robotic arm, as well as the joint coordinate system of each joint according to the rules of the world coordinate system.

The coordinate system origin $F_0$ of the reference coordinate system $F_0$-$X_0Y_0Z_0$ is fixedly connected to the base of the robotic arm, the $Z_0$ axis points from point $F_0$ to the translating joint 1, the $Y_0$ axis points from point $F_0$ of the base to the serial robotic arm, and the pointing of $X_0$ axis conforms to the right-hand coordinate system.

The origin $L_1$ of the joint coordinate system $L_1$-$X_1Y_1Z_1$ of the translating joint 1 is fixedly connected to the translating joint 1, and the pointing direction of each coordinate axis is the same as the corresponding axis of the reference coordinate system.

The origin $R_2$ of the joint coordinate system $R_2$-$X_2Y_2Z_2$ of the rotating joint 2 is fixedly connected to the rotating joint 2 and coincides with $L_1$. The $Z_2$ and $Z_1$ axes point in the same direction, the $X_2$ axis points in the opposite direction to the $X_1$ axis, and the $Y_2$ axis points in the opposite direction to the $Y_1$ axis.

The origin $L_3$ of the joint coordinate system $L_3$-$X_3Y_3Z_3$ of the translating joint 3 is fixedly connected to the translating joint 3, the $Z_3$ axis points from point $L_1$ to point $L_3$, and the $X_3$ axis points in the same direction as the $X_2$ axis, and the $Y_3$ axis points in the same direction as the $Z_2$ axis.

The origin $R_4$ of the joint coordinate system $R_4$-$X_4Y_4Z_4$ of the rotating joint 4 is fixedly connected to the rotating joint 4 and coincides with point $L_3$ (in Figure 4, in order to clearly illustrate the joint coordinate system of the translating joint 3 and the joint coordinate system of the rotating joint 4, $L_3$ and $R_4$ are labeled separately, and the same below). The $Z_4$ axis points in the opposite direction to the $Y_3$ axis. Initially, the $X_4$ axis points in the opposite direction to the $X_3$ axis, and the $Y_4$ axis points in the opposite direction to the $Z_3$ axis.

The origin $R_5$ of the joint coordinate system $R_5$-$X_5Y_5Z_5$ of the rotating joint 5 is fixedly connected to the rotating joint 5. The directions of the $Z_5$ axis and the $Z_4$ axis are the same. Initially, the $X_5$ axis points in the opposite direction to the $X_4$ axis , and the $Y_5$ axis points in the opposite direction to the $Y_4$ axis.

The origin $L_6$ of the joint coordinate system $L_6$-$X_6Y_6Z_6$ of the translating joint 6 is fixedly connected to the translating joint 6. The $Z_6$ axis points from point $R_5$ to point $L_6$, the $X_6$ axis points in the same direction as the $X_5$ axis and the $Y_6$ axis points in the same direction as the $Z_5$ axis.

The origin $R_7$ of the joint coordinate system $R_7$-$X_7Y_7Z_7$ of the rotating joint 7 is fixedly connected to the rotating joint 7 and coincides with point $L_6$. Initially, the $Z_7$ axis points in the opposite direction to the $Y_6$ axis the $Y_7$ axis points in the opposite direction to the $X_6$ axis, and the $X_7$ axis and $Z_6$ axis point in the same direction.

The origin $L_8$ of the joint coordinate system $L_8$-$X_8Y_8Z_8$ of the translating joint 8 is fixedly connected to the translating joint 8. The $Z_8$ axis points from point $L_8$ to point $R_7$, the $X_8$ axis points in the same direction as the $X_7$ axis, and the $Y_8$ axis points in the same direction as the $Y_7$ axis.

The origin $R_9$ of the joint coordinate system $R_9$-$X_9Y_9Z_9$ of the rotating joint 9 is fixedly connected to the rotating joint 9 and coincides with point $L_8$. The direction of the $Z_9$ axis is opposite to the direction of the $Y_8$ axis. Initially, the direction of the $X_9$ axis is opposite to the direction of the $Z_8$ axis, and the direction of the $Y_9$ axis is the same as the direction of the $X_8$ axis.

The origin $L_{10}$ of the joint coordinate system $L_{10}$-$X_{10}Y_{10}Z_{10}$ of the translating joint 10 is fixedly connected to the translating joint 10, the $Z_{10}$ axis points from point $R_9$ to point $L_{10}$, the $X_{10}$ axis points in the same direction as the $X_9$ axis, and the $Y_{10}$ axis points in the same direction as the $Z_9$ axis.

$F_0R_2$ has a length of $l_1$, $R_2R_4$ has a length of $l_2$, $R_4R_5$ has a length of $l_3$, $R_5R_7$ has a length of $l_4$, $R_7R_9$ has a length of $l_5$, $R_9L_{10}$ has a length of $l_6$, points $L_1$, $R_2$, $L_3$, $R_4$, $R_5$, $L_6$ and $R_7$ are all located on the same horizontal plane.

Step 2: Acquiring the DH parameters and calculating the transformation relationship from the reference coordinate system to the end joint of the serial robotic arm.

DH parameters are shown in Table 1. In Table 1, in order to avoid confusion of the rotation angles 0 and $2\pi$ of the rotating joints, the rotation angle of the two positions of 0 and $2\pi$ for the Z axis are avoided in the DH parameters.

Table 1 DH parameter table of the serial robotic arm shown in Figure 4

| Serial number / Item | 0 | 1 | 2 | 3 | 4 | 5 | 6 | 7 | 8 | 9 | 10 |
|---|---|---|---|---|---|---|---|---|---|---|---|
| Translation $d_i$ along Z axis | - | $d_1$ | 0 | $d_3$ | 0 | 0 | $d_6$ | 0 | $d_8$ | 0 | $d_{10}$ |
| Rotation $\theta_i$ about Z axis | - | 0 | $\pi+\theta_2$ | 0 | $\pi+\theta_4$ | $\pi+\theta_5$ | 0 | $\frac{\pi}{2}+\theta_7$ | 0 | $-\frac{\pi}{2}+\theta_9$ | 0 |
| Rotation $\alpha_i$ about X axis | - | 0 | 0 | $\frac{\pi}{2}$ | $\frac{\pi}{2}$ | 0 | $\frac{\pi}{2}$ | $\frac{\pi}{2}$ | 0 | $\frac{\pi}{2}$ | $\frac{\pi}{2}$ |
| Translation $a_i$ along X axis | - | 0 | 0 | 0 | 0 | $a_4$ | 0 | 0 | 0 | 0 | 0 |

The transformation matrix between the Stewart calculation coordinate system and the reference coordinate system is solved, that is,
the transformation matrix and its inverse matrix between the translating joint coordinate system $L_{10}$-$X_{10}Y_{10}Z_{10}$ and the reference coordinate system $F_0$- $X_0Y_0 Z_0$ can be obtained.

According to the direct kinematics of the robot, the transformation matrix between two adjacent joints is:

$$T_i = \begin{bmatrix} \cos\theta_i & -\sin\theta_i & 0 & a_{i-1} \\ \sin\theta_i \times \cos\alpha_{i-1} & \cos\theta_i \times \cos\alpha_{i-1} & -\sin\alpha_{i-1} & -d_i \times \sin\alpha_{i-1} \\ \sin\alpha_{i-1} \times \sin\theta_i & \sin\alpha_{i-1} \times \cos\theta_i & \cos\alpha_{i-1} & d_i \times \cos\alpha_{i-1} \\ 0 & 0 & 0 & 1 \end{bmatrix}$$

The transformation matrix from the m-th joint (containing degrees of freedom of the m j oint) to the N-th joint can be expressed as:

$$_{n}^{m}T = {}_{m+1}^{m}T \cdot {}_{m+2}^{m+1}T \cdots {}_{n-1}^{n-2}T \cdot {}_{n}^{n-1}T$$

The transformation matrix $_{10}^{0}T$ of single serial robotic arm from the base to the static platform of the Stewart platform can be obtained through solving, that is, the transformation matrix from the reference coordinate system to the Stewart calculation coordinate system, which is named $T_{\text{trans\_mach\_st}}$, and the transformation matrix from the Stewart calculation coordinate system to the reference coordinate system is the inverse matrix $T_{\text{trans\_mach\_st}}^{-1}$.

Step 3: Performing coordinate transformation between the reference coordinate system and the Stewart-calculation coordinate system according to the transformation matrix and the inverse matrix of the transformation matrix.

In embodiments of the present application, the user coordinate system is established to simplify the motion mapping of the master-slave control. In the case that the robotic arm system works with a single arm, the master-slave mapping method for parallel platform in this optional embodiment further includes the following steps.

Step S205: Establishing a slave user coordinate system, wherein the X-Y coordinate plane of the slave user coordinate system is parallel to the X-Y plane of the reference coordinate system, and the origin of the slave user coordinate system coincides with the origin of the calculation coordinate system.

Step S206: Acquiring the viewing angle value input by the user, and determining the transformation relationship between the slave user coordinate system and the reference coordinate system according to the viewing angle value and the transformation relationship between the reference coordinate system and the calculation coordinate system.

The rotation angle about the Z axis of the X-Y coordinate plane of the slave user coordinate system established in step S205 is the viewing angle value. When working with a single arm, the viewing angle value is input by the user according to the viewing angle, which is named $\theta_{theta\_mach\_user}$. According to the definition of user coordinates, the transformation matrix of the slave user coordinate system relative to the reference coordinate system can be obtained:

$$T_{trans\_mach\_user} = \begin{bmatrix} \cos(\theta_{theta\_mach\_user}) & -\sin(\theta_{theta\_mach\_user}) & 0 & {}^{0}_{10}T(1,4) \\ \sin(\theta_{theta\_mach\_user}) & \cos(\theta_{theta\_mach\_user}) & 0 & {}^{0}_{10}T(2,4) \\ 0 & 0 & 0 & {}^{0}_{10}T(3,4) \\ 0 & 0 & 0 & 1 \end{bmatrix}$$

Where, ${}^{0}_{10}T(1,4)$ indicates the data in the 1st row and 4th column in the above ${}^{0}_{10}T$.

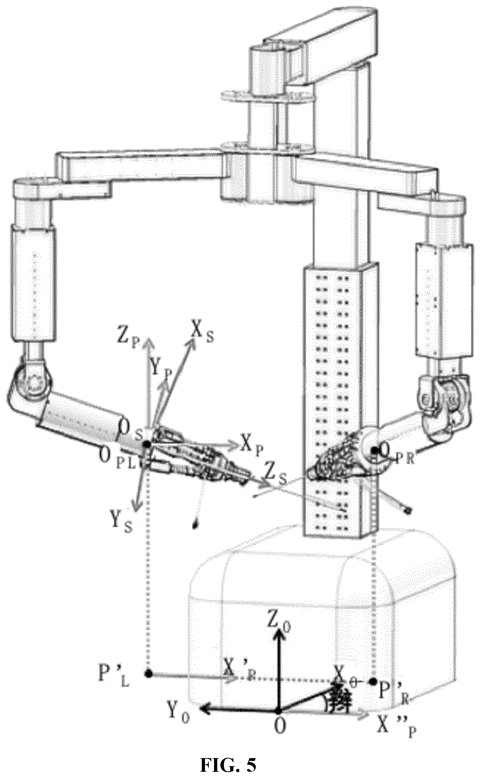

FIG. 5 is a schematic diagram of the slave user coordinate system in case that the robotic arm system provided by the optional embodiment of the present application work with two arms. As shown in FIG. 5, the reference coordinate system is $O_0\text{-}X_0Y_0Z_0$, the Stewart calculation coordinate system is $Os\text{-}XsYsZs$, and the slave user coordinate system is $O_P\text{-}X_PY_PZ_P$.

In the case that the robotic arm system works with two arms, the master-slave mapping method for parallel platform according to this optional embodiment further includes the following steps.

Step S207: establishing the first slave user coordinate system of the first serial robotic arm and the second slave user coordinate system of the second serial robotic arm, wherein the X-axis direction of the first slave user coordinate system is the same as and colinear with the X-axis direction of the second slave user coordinate system, the origin of the first slave user coordinate system coincides with the origin of the calculation coordinate system of the first serial robotic arm, and the origin of the second slave user coordinate system coincides with the origin the calculation coordinate system of the second serial robotic arm.

Taking the reference coordinate system as the reference, and the direction facing the object to be operated as the forward directions of the serial robotic arms, at the left-hand side, it is the left serial robotic arm, and at the right-hand side, it is the right serial robotic arm. The DH parameters of the left and right arms are input respectively, according to the single-arm transformation matrix setting method, and the respective transformation matrices $T_{trans\_mach\_st\_left}$ and $T_{trans\_mach\_st\_right}$ of the left and right arms can be obtained. The coordinates of the origin of the static platform of the two serial robotic arms in the reference coordinate system are:

$$C_{coord\_mach\_st\_left} =$$
$$[T_{trans\_mach\_st\_left}(1,4) \quad T_{trans\_mach\_st\_left}(2,4) \quad T_{trans\_mach\_st\_left}(3,4) \quad 1]^{T};$$

$$C_{coord\_mach\_st\_right} =$$
$$[T_{trans\_mach\_st\_right}(1,4) \quad T_{trans\_mach\_st\_right}(2,4) \quad T_{trans\_mach\_st\_right}(3,4) \quad 1]^{T}.$$

Here, it is stipulated that the directions of the X axes of the slave user coordinate systems of the two serial robotic arms are the same and collinear, and the positive direction is the direction pointing from the point $C_{coord\_mach\_st\_left}$ to the point $C_{coord\_mach\_st\_right}$.

Step S208: determining the angle between the X-axis direction of the first slave user coordinate system and the X-axis direction of the reference coordinate system, and determining the second transformation relationship between the reference coordinate system and the first slave user coordinate system and the second transformation relationship between the reference coordinate system and the second slave user coordinate system according to the angle and the transformation relationships between the reference coordinate system and the calculation coordinate system of each serial robotic arm.

The angle $\theta_{theta\_mach\_user}$ between the X axes under the first slave user coordinate system and the reference coordinate system can be calculated by the following equation:

$$\theta_{theta_{mach_{user}}} = \text{atan2}(C_{coord\_mach\_st\_right}(2)$$
$$- C_{coord\_mach\_st\_left}(2), C_{coord\_mach\_st\_right}(1)$$
$$- C_{coord\_mach\_st\_left}(1))$$

Where, θ=atan2(y,x), which is a built-in function in MATLAB or C language library. Therefore, the transformation matrix of the slave user coordinate system corresponding to the left or right serial robotic arm can be obtained by a method similar to the single-arm transformation matrix:

$$T_{trans\_mach\_user\_left}$$
$$= \begin{bmatrix} \cos(\theta_{theta\_mach\_user}) & -\sin(\theta_{theta\_mach\_user}) & 0 & C_{coord\_mach\_st\_left}(1) \\ \sin(\theta_{theta\_mach\_user}) & \cos(\theta_{theta\_mach\_user}) & 0 & C_{coord\_mach\_st\_left}(2) \\ 0 & 0 & 0 & C_{coord\_mach\_st\_left}(3) \\ 0 & 0 & 0 & 1 \end{bmatrix}$$

$$T_{trans\_mach\_user\_right}$$
$$= \begin{bmatrix} \cos(\theta_{theta\_mach\_user}) & -\sin(\theta_{theta\_mach\_user}) & 0 & C_{coord\_mach\_st\_right}(1) \\ \sin(\theta_{theta\_mach\_user}) & \cos(\theta_{theta\_mach\_user}) & 0 & C_{coord\_mach\_st\_right}(2) \\ 0 & 0 & 0 & C_{coord\_mach\_st\_right}(3) \\ 0 & 0 & 0 & 1 \end{bmatrix}$$

The transformation matrix $T_{trans\_mach\_user}$ of the slave user coordinate system relative to the reference coordinate system is obtained, and thus the transformation matrix $T^{-1}_{trans\_mach\_st}$ of the reference coordinate system relative to the slave user coordinate system can be obtained.

In the case that the transformation matrix of the reference coordinate system to the slave user coordinate system and transformation matrix of the reference coordinate system to the Stewart coordinate system have been obtained, the transformation matrix $T_{trans\_user\_st}$ from the slave user coordinate system to the Stewart calculation coordinate system can be calculated by the following equation:

$$T_{trans\_user\_st} = T_{trans\_mach\_user}^{-1} \cdot T_{trans\_mach\_st}.$$

The transformation matrix from the Stewart calculation coordinate system to the slave user coordinate system is the inverse matrix $T^{-1}_{trans\_user\_st}$.

In the above embodiment, when the robotic arm system works with a single arm, the transformation relationship between the slave user coordinate system and the reference coordinate system is determined by the viewing angle value input by the user. When the robotic arm system works with two arms, because the slave user coordinate system is established for each single arm, after the X-axis directions of the two slave user coordinate systems have been set to be the same and collinear, the angle between the X-axis direction of the slave user coordinate systems and the X-axis direction of the reference coordinate system can be determined as the viewing angle value, and the transformation relationship between the slave user coordinate system of each single arm and the reference coordinate system is then determined.

After obtaining the transformation relationship between the slave user coordinate system and the reference coordinate system, the transformation relationship between the slave user coordinate system and the calculation coordinate system of the serial robotic arm can be determined according to the transformation relationship between the reference coordinate system and the calculation coordinate system, and the transformation relationship between the slave user coordinate system and the reference coordinate system.

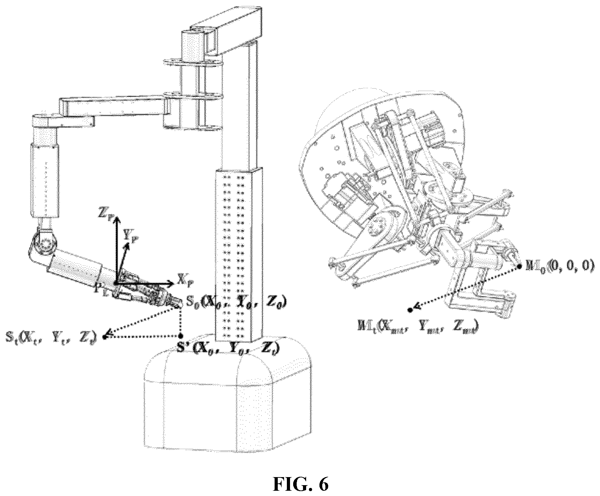

FIG. 6 is a schematic diagram of a master-slave mapping method for parallel platform according to an optional embodiment of the present application. As shown in FIG. 6, the left picture shows a slave manipulator, and the right picture shows a master manipulator. Referring to FIG. 6, specifically, the master-slave mapping method for parallel platform provided by this optional embodiment may include the following steps.

Step 1: Regarding a time period from the moment an operator holds the master manipulator until the operator's hand leaves the master manipulator as a working cycle T. The time when the operator holds the master manipulator and starts to operate is time T(0), and the position coordinates of the master manipulator at this time are set as the origin M0(0, 0, 0); the position coordinates of an end point of an operating instrument mounted on the movable platform in the slave user coordinate system at this time are S0(X0, Y0, Z0), the system calculates and saves S0(X0, Y0, Z0) as known values, denoted as $C_{coord\_now\_user}$

Step 2: Setting the position coordinates of the end point of the master manipulator as $M_t(X_{mt}, Y_{mt}, Z_{mt})$ at any time t in the working cycle, denoted as

$$C_{coord\_offset\_mas}:$$
$$C_{coord\_offset\_mas} = \begin{bmatrix} {}^{C}\!x_{coord\_offset\_mas} & {}^{C}\!y_{coord\_offset\_mas} & {}^{C}\!z_{coord\_offset_{mas}} & 1 \end{bmatrix}$$

At this time, the position coordinates $S_t(X_t, Y_t, Z_t)$ of the motion target point of the end point of the instrument in the slave user coordinate system can be obtained by using $M_t(X_{mt}, Y_{mt}, Z_{mt})$ and a displacement proportional scaling coefficient $K_0$:

$$X_t = X_0 + K_0 * X_{mt};$$

$$Y_t = Y_0 + K_0 * Y_{mt};$$

$$Z_t = Z_0 + K_0 * Z_{mt}.$$

Based on the fact that the position coordinates of the end point of the instrument in the slave user coordinate system at the T(0) moment are $S_0(X_0, Y_0, Z_0)$, in each motion performing cycle, the master manipulator will send the current coordinates $M_t(X_{mt}, Y_{mt}, Z_{mt})$ to solve the coordinates $S_t(X_t, Y_t, Z_t)$ of the end point of the instrument in the slave user coordinate system at this time, which is denoted as $C_{coord\_new\_user}$: $C_{coord\_new\_user} = C_{coord\_now\_user} + K_0 \times C_{coord\_offset\_mas}$.

Step 3: Transforming the coordinates $S_t(X_t, Y_t, Z_t)$ of the end point of the instrument in the slave user coordinate system to the coordinates in the Stewart calculation coordinate system through the transformation matrix of the slave user coordinate system to the Stewart calculation coordinate system, which is denoted as

$$C_{coord\_new\_st}:$$
$$C_{coord\_new\_st} = T_{trans\_st\_user} \cdot C_{coord\_new\_user}.$$

Where, $T_{trans\_st\_user}$ is the transformation matrix of the slave user coordinate system to the Stewart calculation coordinate system.

Step 4: Solving the motion amount of each joint of the platform through the inverse kinematics of the Stewart platform to complete the master-slave motion mapping as the coordinates of the end point of the instrument in the Stewart calculation coordinate system are known.

In some of the embodiments, the preset displacement proportional coefficient $K_0$ can be adjusted, and this value may be a value greater than 1 or a value less than 1; wherein, when the preset displacement proportional coefficient $K_0$ is a value less than 1, high-precision control of the slave manipulator can be achieved, and the shaking of the operator's hand during the operation can be eliminated to improve the operation reliability.

In the above-mentioned master-slave mapping method, the motion mapping control relative to zero position is adopted, which avoids the accumulation of errors and improves the control precision and operation safety.

This embodiment also provides a robotic arm system including a parallel platform, a master manipulator, a memory, and a controller. The parallel platform includes a static platform, a movable platform, and a plurality of telescopic rods arranged between the static platform and the movable platform. A computer program is stored in the memory, and the controller is configured to run the computer program to execute any one of the master-slave mapping methods for parallel platform in the above-mentioned embodiments.

In addition, in combination with the methods in the foregoing embodiments, the embodiments of the present application further provide a computer-readable storage medium for implementation. Computer program instructions are stored on the computer-readable storage medium. When the computer program instructions are executed by a processor, any one of the master-slave mapping methods for parallel platform in the above-mentioned embodiments is implemented.

It should be noted that, for specific examples in this embodiment, reference may be made to the examples described in the foregoing embodiments and optional examples, and details are not described herein again in this embodiment.

The technical features of the above-described embodiments can be combined arbitrarily. For the sake of brevity, not all possible combinations of the technical features in the above-described embodiments are described. However, as long as there is no conflict between the combinations of these technical features, all should be regarded as the scope described in this specification.

The above-mentioned embodiments only represent several embodiments of the present application, and the descriptions thereof are relatively specific and detailed, but should not be construed as a limitation on the scope of the patent application. It should be pointed out that for those skilled in the art, without departing from the concept of the present application, several modifications and improvements can be made, which all belong to the protection scope of the present application. Therefore, the scope of protection of the patent of the present application shall be subject to the appended claims.

**Claims**

1. A master-slave mapping method for parallel platform applied to a robotic arm system, the robotic arm system comprising a parallel platform and a master manipulator, the parallel platform comprising a static platform, a movable platform, and a plurality of telescopic rods arranged between the static platform and the movable platform, wherein the master-slave mapping method for parallel platform comprises steps of:

   establishing a calculation coordinate system of the parallel platform on the static platform, establishing a slave user coordinate system on the static platform, and establishing a master user coordinate system on the master manipulator, wherein an origin of the calculation coordinate system coincides with an origin of the slave user coordinate system, and Z axes of the slave user coordinate system and the master user coordinate system are both parallel to a Z axis of a reference coordinate system;
   acquiring a first transformation relationship between the slave user coordinate system and the calculation coordinate system;
   mapping displacement amounts of an end of the master manipulator in the master user coordinate system to displacement amounts of an end of the movable platform in the slave user coordinate system according to a set proportional coefficient, to obtain a first target position of the end of the movable platform in the slave user coordinate system;
   determining a second target position of the end of the movable platform in the calculation coordinate system according to the first transformation relationship and the first target position; and
   obtaining a movement amount of each of the telescopic rods of the parallel platform by using an inverse kinematics algorithm according to the second target position, and controlling a motion of the parallel platform according to the movement amount of each of the telescopic rods.

2. The master-slave mapping method for parallel platform according to claim 1, wherein the step of acquiring the first transformation relationship between the slave user coordinate system and the calculation coordinate system comprises:

   acquiring a second transformation relationship between the reference coordinate system and the slave user coordinate system, and acquiring a third transformation relationship between the reference coordinate system and the calculation coordinate system; and
   determining the first transformation relationship according to the second transformation relationship and the third transformation relationship.

3. The master-slave mapping method for parallel platform according to claim 2, wherein the reference coordinate system is a base coordinate system of the robotic arm system; and the robotic arm system further comprises a serial robotic arm, and the parallel platform is mounted on an end of the serial robotic arm; wherein, acquiring a third transformation relationship between the reference coordinate system and the calculation coordinate system comprises:

establishing a joint coordinate system of each joint in the serial robotic arm;

acquiring DH parameters between a joint coordinate system of a first joint in the serial robotic arm and the reference coordinate system, and determining a transformation relationship $^{0}_{1}T$ between the reference coordinate system and the joint coordinate system of a first joint, wherein the first joint is a joint directly connected to the base, and the DH parameters are traditional DH parameters or improved DH parameters;

acquiring the DH parameters between the joint coordinate system of the i-th joint and the joint coordinate system of the (i-1)-th joint in the serial robotic arm, and determining a transformation relationship $^{i-1}_{i}T$ between the joint coordinate system of the (i-1)-th joint and the joint coordinate system of the i-th joint, where i=2, 3, 4,...,N; N is a total number of joints of the serial robotic arm;

determining a transformation relationship $^{0}_{N}T$ between the reference coordinate system and the joint coordinate system of the N-th joint according to the transformation relationship $^{0}_{1}T$ and the transformation relationship $^{i-1}_{i}T$, which is the third transformation relationship, wherein the joint coordinate system of the N-th joint coincides completely with the calculation coordinate system.

4. The master-slave mapping method for parallel platform according to claim 3, wherein the serial robotic arm comprises rotating joints and translating joints, wherein a Z axis of the joint coordinate system of a rotating joint is set along a rotation axis, and a Z axis of the joint coordinate system of a translating joint is set along a moving direction; the reference coordinate system and the joint coordinate system of each joint are all left-handed system or right-handed system, and when a joint before a rotating joint is a translating joint, an origin of the joint coordinate system of the rotating joint coincides with an origin of the joint coordinate system of the translating joint.

5. The master-slave mapping method for parallel platform according to claim 4, wherein a rotation angle of a Z axis in the DH parameters of the rotating joints in the serial robotic arm is not 0 or $2\pi$.

6. The master-slave mapping method for parallel platform according to claim 2, wherein acquiring a second transformation relationship between the reference coordinate system and the slave user coordinate system comprises:

acquiring a viewing angle value of a user relative to the robotic arm system;

determining the second transformation relationship between the reference coordinate system and the slave user coordinate system according to the viewing angle value and the third transformation relationship.

7. The master-slave mapping method for parallel platform according to claim 6, wherein the viewing angle value is determined based on configuration information input by the user in the case that the robotic arm system operates with a single arm.

8. The master-slave mapping method for parallel platform according to claim 2, wherein in the case of a first serial robotic arm and a second serial robotic arm working simultaneously in the robotic system, acquiring a second transformation relationship between the reference coordinate system and the slave user coordinate system comprises:

establishing a first slave user coordinate system of the first serial robotic arm and a second slave user coordinate system of the second serial robotic arm, wherein a X-axis direction of the first slave user coordinate system is the same as and collinear with a X-axis direction of the second slave user coordinate system, and an origin of the first slave user coordinate system coincides with an origin of the calculation coordinate system of the first serial robotic arm, and an origin of the second slave user coordinate system coincides with an origin of the calculation coordinate system of the second serial robotic arm;

determining an angle between the X-axis direction of the first slave user coordinate system and the X-axis direction of the reference coordinate system, and determining the second transformation relationship between the reference coordinate system and the first slave user coordinate system and the second transformation relationship between the reference coordinate system and the second slave user coordinate system, according to the angle and transformation relationship between the reference coordinate system and the calculation coordinate system of each serial robotic arm.

9. A robotic arm system, comprising a parallel platform, a master manipulator, a memory, and a controller, the parallel platform comprising a static platform, a movable platform, and a plurality of telescopic rods arranged between the static platform and the movable platforms, wherein a computer program is stored in the memory, and the controller is configured to run the computer program to execute the master-slave mapping method for parallel platform according to any one of claims 1 to 8.

10. The robotic arm system according to claim 9, wherein the robotic arm system further comprises a serial robotic arm, and the parallel platform is mounted on an end of the serial robotic arm.

11. A storage medium, wherein a computer program is stored on the storage medium, wherein the computer program is configured to execute the master-slave mapping method for parallel platform according to any one of claims 1 to 8 when running.

establishing the calculation coordinate system of the parallel platform on the static platform, establishing the slave user coordinate system on the static platform, and establishing the master user coordinate system on the master manipulator — S101

acquiring a first transformation relationship between the slave user coordinate system and the calculation coordinate system — S102

mapping the displacement amount of an end of the master manipulator in the master user coordinate system to an end of the movable platform in the slave user coordinate system according to a set proportional coefficient, to obtain the first target position of the end of the movable platform in the slave user coordinate system — S103

determining the second target position of the end of the movable platform in the calculation coordinate system according to the first transformation relationship and the first target position — S104

obtaining a movement amount of each of the telescopic rods of the parallel platform by using inverse kinematics algorithm according to the second target position, and controlling a motion of the parallel platform according to the movement amount of each of the telescopic rods — S105

**FIG. 1**

establishing a joint coordinate system of each joint in the serial robotic arm — S201

acquiring DH parameters of joint coordinate system of the first joint in the serial robotic arm and the reference coordinate system and determining the transformation relationship between the reference coordinate system and the joint coordinate system of the first joint — S202

acquiring the DH parameters of the joint coordinate system of the i-th joint and the joint coordinate system of the (i-1)-th joint in the serial robotic arm, and determining the transformation relationship between the joint coordinate system of the (i-1)-th joint and the joint coordinate system of the i-th joint — S203

determining the transformation relationship between the reference coordinate system and the joint coordinate system of the N-th joint according to the transformation relationship and the transformation relationship — S204

**FIG. 2**

**FIG. 3**

**FIG. 4**

**FIG. 5**

**FIG. 6**

## INTERNATIONAL SEARCH REPORT

| | International application No. |
|---|---|
| | **PCT/CN2020/141274** |

**A. CLASSIFICATION OF SUBJECT MATTER**

B25J 9/00(2006.01)i

According to International Patent Classification (IPC) or to both national classification and IPC

**B. FIELDS SEARCHED**

Minimum documentation searched (classification system followed by classification symbols)

B25J

Documentation searched other than minimum documentation to the extent that such documents are included in the fields searched

Electronic data base consulted during the international search (name of data base and, where practicable, search terms used)

CNPAT, CNKI, WPI, EPODOC, IEEE: 机械臂, 主从, 逆, 坐标系, master, slave, mechanical arm, coordinate system, inverse

**C. DOCUMENTS CONSIDERED TO BE RELEVANT**

| Category* | Citation of document, with indication, where appropriate, of the relevant passages | Relevant to claim No. |
|---|---|---|
| A | CN 109968310 A (CHONGQING YUBOCHUANG INTELLIGENT EQUIPMENT RESEARCH INSTITUTE CO., LTD.) 05 July 2019 (2019-07-05) description, paragraphs [0006]-[0050], figure 1 | 1-11 |
| A | CN 107589934 A (DALIAN UNIVERSITY OF TECHNOLOGY) 16 January 2018 (2018-01-16) entire document | 1-11 |
| A | CN 107374727 A (CHONGQING JINSHAN MEDICAL INSTRUMENT CO., LTD.) 24 November 2017 (2017-11-24) entire document | 1-11 |
| A | CN 107662195 A (DONGFANG ELECTRIC CORPORATION) 06 February 2018 (2018-02-06) entire document | 1-11 |
| A | US 5053975 A (HITACHI, LTD.) 01 October 1991 (1991-10-01) entire document | 1-11 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **08 September 2021** | **28 September 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/141274**

| Patent document cited in search report | | | Publication date (day/month/year) | Patent family member(s) | | | Publication date (day/month/year) |
|---|---|---|---|---|---|---|---|
| CN | 109968310 | A | 05 July 2019 | None | | | |
| CN | 107589934 | A | 16 January 2018 | None | | | |
| CN | 107374727 | A | 24 November 2017 | None | | | |
| CN | 107662195 | A | 06 February 2018 | None | | | |
| US | 5053975 | A | 01 October 1991 | CA | 1330364 | C | 21 June 1994 |
| | | | | DE | 68927138 | D1 | 17 October 1996 |
| | | | | JP | H01310873 | A | 14 December 1989 |
| | | | | EP | 0345813 | A2 | 13 December 1989 |

Form PCT/ISA/210 (patent family annex) (January 2015)